# EUROPEAN PATENT APPLICATION

(11) **EP 4 311 573 A1**
(43) Date of publication of application: **31.01.2024**
(21) Application number: 22187526.3
(22) Date of filing: 28.07.2022
(51) Int. Cl.: A61N 5/10

(54) **METHOD AND SYSTEM FOR DETERMINING A TREATMENT CONTINUATION INFORMATION FOR A RADIOTHERAPY**

(71) Applicant: Siemens Healthcare GmbH, 91052 Erlangen (DE)
(72) Inventor: Nioutsikou, Elena, 91056 Erlangen (DE); Tasler, Martin, 91054 Erlangen (DE)
(74) Representative: Siemens Healthineers Patent Attorneys

(57) **Abstract**

Method for determining a treatment continuation information for a radiotherapy of a tumor target of a patient, comprising the steps:
- Receiving (S3) an initial treatment plan (Pᵢ) for a radiotherapy of the patient, wherein the initial treatment plan (Pᵢ) is based on first medical image data (Dₜ₁) of tumor target,
- Receiving (S2) second medical image data (Dₜ₂) of the tumor target, wherein the second medical image data (Dₜ₂) are acquired a first treatment time period after acquiring the first medical image data (Dₜ₁),
- Determining (S4) a treatment responding information (I_{TR}) based on the second medical image data (Dₜ₂) and on supplementary data (S), wherein the supplementary data (S) comprise the first medical image data (Dₜ₁), medical image data acquired between the first and second medical image data (Dₜ₁, Dₜ₂), the initial treatment plan (Pᵢ) and/or an initial tumor target information,
- Determining (S5) the treatment continuation information based on the treatment responding information (I_{TR}) and on the initial treatment plan (Pᵢ),
- Providing (S6) the treatment continuation information.

## Description

The present invention relates to a method and system for determining a treatment continuation information for a radiotherapy of a tumor target of a patient. Furthermore, the invention relates to a computer programme and a machine readable medium for determining a treatment continuation information for a radiotherapy.

Radiotherapy, also named radiation therapy, is used to treat cancer and other diseases with ionizing radiation. Conventional radiotherapy systems generate and direct a beam of radiation to a targeted treatment volume within a patient. The radiation beam is intended to injure or destroy cells within the target volume by causing ionizations within the cells or other radiation-induced cell damage.

Radiotherapy is carried out based on treatment plans. The treatment plans are intended for delivering radiation to a patient, wherein radiation delivered to a target area is maximized and the radiation delivered to surrounding healthy tissue is minimized. In this regard, several different techniques have been developed to address different target areas and types of tumors, as well as radiation exposure concerns

Recent advances in fractionated external beam radiation therapy, such as three-dimensional conformal and intensity-modulated radiation therapy (IMRT), have increased the ability to deliver radiation doses that conform tightly to a target volume. This tight conformance results in steep dose gradients inside the volume. For example, IMRT can create a dose gradient of 10% mm⁻¹ inside a target volume.

A treatment plan is designed assuming that a target volume will be in a particular position relative to a beam source during treatment. If the target volume is not positioned exactly as assumed by the treatment plan, the steep gradient may occur within sensitive healthy tissue surrounding the volume causing destruction of healthy tissue while sparing some malignant tissue. Thus, it is increasingly important to precisely position the target volume with respect to the beam source.

It is not unusual for the target volume to change position or shape within the patient (e.g., to translate along one or more axes and/or rotate about one or more axes) after a treatment plan is designed but prior to performing the treatment.

In order to simulate the current location of the target volume with respect to the external beams, three-dimensional imaging of the patient is often provided prior to treatment delivery. Systems attempting to provide such imaging include: (1) a "CT on rails'' system, requiring an additional diagnostic computed tomography machine in the treatment room; (2) a kilovoltage cone beam CT (kVCBCT) system, consisting of an additional kilovoltage X-ray source and detector attached to a treatment gantry; (3) a megavoltage cone beam CT (MVCBCT) system using the pre-existing treatment machine and an EPID for imaging; (4) a MVCT system, using the pre-existing treatment machine with an attached arc of detectors; (5) a tomotherapy system, replacing the traditional treatment machine with a CT ring and a MV beam source; (6) a pre-treatment magnetic resonance imaging (MRI) of the patient; (7) a pre-treatment PET/CT imaging.

In known systems for patient radiotherapy treatment, 3D X-Ray images are taken to plan a treatment process. Subsequent images (of which many dozens may be taken) may be used to position the patient but are typically not used to re-plan and refine the treatment process, nor are subsequent images used to incrementally or in ensemble, determine anatomical or functional changes with respect to an initial planning image.

A typical radiotherapy treatment plan is done at the beginning of treatment and as such it is not representative of the changes that may occur during the course of treatment. Even if additional images are taken during treatment, these are not accompanied by information that reveals the (perceived) efficiency of the treatment nor its full effect (e.g., that the tumor is necrotic). These images however contain information about the patient's response to the current treatment. Consequently, no support exists today for the user to quickly take a decision on whether treatment adaptation, treatment course alteration, treatment boosting, continuing or cessation is optimal for the patient under consideration.

This results in potentially ineffective or impaired radiotherapy treatment. The present inventors have realized these problems and inefficiencies. Accordingly, a method and systems to determine a treatment continuation information for a radiotherapy of a patient are desired. A system according to invention principles addresses these deficiencies and related problems.

The present invention concerns a method for determining a treatment continuation information for a radiotherapy. Determining the treatment continuation information can optionally comprises generating a treatment plan for a radiotherapy, especially of a tumor target of a patient. The method for determining a treatment continuation information for a radiotherapy comprises the steps:
- Receiving an initial treatment plan for a radiotherapy of the patient, wherein the initial treatment plan is based on first medical image data of the patient, wherein the first medical image data comprise the tumor target,
- Receiving second medical image data of the patient, wherein the second medical image data comprise the tumor target, wherein the second medical image data are acquired a first treatment time period after the first medical image data,

- Determining a treatment responding information based on the second medical image data and supplementary data, wherein the supplementary data comprise the first medical image data, medical image data acquired between the first and second medical image data, the initial treatment plan and/or an initial tumor target information,
- Determining the treatment continuation information based on the treatment responding information and on the initial treatment plan, wherein the treatment continuation information comprises an information and/or suggestion for a further treatment of the patient and/or further radiotherapy of the patient,
- Providing the treatment continuation information.

The method is preferably configured as a computer-implemented method. Especially, the steps of the method can be performed by a computer or processor, in particular, the method and/or steps are performed by a system for determining a treatment continuation information, particularly to determine a treatment plan. Especially, the steps of the method can be performed on at least two systems, computers, or processors, wherein the systems, computers or processors are connected for a data exchange and/or collaboration. A processor as used herein is a device and/or set of machine-readable instructions for performing tasks. A processor comprises any one or combination of, hardware, firmware, and/or software. A processor acts upon information by manipulating, analyzing, modifying, converting, or transmitting information for use by an executable procedure or an information device, and/or by routing the information to an output device. A processor may use or comprise the capabilities of a controller or microprocessor, for example. A processor may be coupled (electrically and/or as comprising executable components) with any other processor enabling interaction and/or communication there-between.

The initial treatment plan is received. E.g., the initial treatment plan is provided by a user, a system and/or via an interface. The interface can be configured as a user interface (UI), especially comprising one or more display images. The UI enables user interaction with a processor or other device and associated data acquisition and processing functions.

The initial treatment plan, preferably also the treatment plan determined according to the invention, is a personalized treatment plan. In other words, the treatment plan and/or the initial treatment plan is a patient specific treatment plan. The initial treatment plan is based on first medical image data. The first medical image data are acquired prior to the radiotherapy. The first medical image data can comprise unprocessed and/or processed (e.g., labeled, or segmented) medical images. The first medical image data can comprise MR (magnetic resonance), CT (computed tomography), X-ray, PET (positron emission tomography) and/or ultrasound images. The first medical image data represent anatomical volumes and determine initial targeting of radiotherapy for cancer treatment. In other words, the first medical image data comprise, represent and/or show an anatomical region of the patient, wherein the tumor target is in this anatomical region. The first medical image data can comprise 2D-, 3D and/or 4D (3D + time) images. Preferably, the first medical image data comprise a plurality of medical images, especially acquired by different modalities (e.g., MR and CT), wherein the images are preferably partly registered.

The initial treatment plan, especially the later determined treatment plan, comprises parameters. The comprised parameters are preferably parameters which define and/or influence the radiotherapy. The initial treatment plan and/or the determined treatment plan comprises for example parameters for the radiotherapy device, e.g., for a LINAC (linear accelerator). The radiotherapy and/or the treatment plan is preferably for a linear accelerator-based three-dimensional conformal radiotherapy (Linac-3DCRT), especially for helical, direct-3DCRT, and/or direct-intensity-modulated radiotherapy (direct-IMRT). The initial treatment plan and/or the determined treatment plan can comprise as parameters a dose prescription for the tumor target and/or fractions of the dose, contour of target, isocenter location and different dose-volume parameters for the tumor target and organs at risk (OARs). Furthermore, parameters of the treatment plan and/or the initial treatment plan may comprise beam angles, dose rate, energy and/or energy distribution of the beam, collimator position, couch position and/or angle, field and/or beam width, modulation factor and/or percentage of leaf open time. For further parameters and/or definition it is referred to Chang, Kyung Hwan, et al. "Statistical Analysis of Treatment Planning Parameters for Prediction of Delivery Quality Assurance Failure for Helical Tomotherapy." (Technology in Cancer Research & Treatment 19 (2020)) and Goswami, Brijesh, et al. "Comparison of Treatment Planning Parameters of Different Radiotherapy Techniques for Craniospinal Irradiation." (Iranian Journal of Medical Physics 18.3 (2021)).

The second medical image data are received for example via an interface, especially a user interface. The second medical data are for example provided by a medical imaging device, wherein the medical imaging device comprises or is configured as a magnetic resonance tomography scanner, a computed tomography scanner, a positron emission tomography scanner, X-ray scanner or ultrasound scanner. In particular, the receiving of the second medical image data comprises an acquiring of the second medical image data and/or the acquiring of the second medical image data is prior to the step of receiving the second medical image data, wherein the acquired second medical image data are received in the receiving step.

The second medical image data comprise, represent and/or show the tumor target, especially the anatomical region of the tumor target. In particular, under the wording medical image data of the tumor target, especially the first and/or second medical image data, medical image data comprising and/or showing the tumor target including neighboring organs at risk are described and/or meant. The second medical image data can be configured and/or acquired using the same imaging modality (e.g., MR or CT) as the first medical image data. The second medical image data can comprise unprocessed and/or processed (e.g., labeled, or segmented) medical images. The second medical image data can comprise MR (magnetic resonance), CT (computed tomography), X-ray, PET (positron emission tomography) and/or ultrasound images. The second medical image data represent anatomical volumes and show the initial targeting of radiotherapy for cancer treatment. In other words, the second medical image data comprise, represent and/or show an anatomical region of the patient, wherein the tumor target is in this anatomical region. The second medical image data can comprise 2D-, 3D and/or 4D (3D + time) images. Preferably, the second medical image data comprise a plurality of medical images, especially acquired by different modalities (e.g., MR and CT), wherein the images are preferably partly registered. The second medical image data can be completely or partly registered with the first medical image data.

The second medical image data are acquired a first treatment time period after the first medical image data. The first treatment time period is preferably greater than one day, especially greater than one week and particularly greater than two weeks. During the first treatment time period at least one treatment of the radiotherapy according to the initial treatment plan and/or a determined treatment plan was applied to the patient. In other words, between acquiring the first medical image data and the second medical image data at least one radiotherapy treatment according to the initial treatment plan was applied to the patient.

The treatment responding information is determined based on the second medical image data and on supplementary data. The determining of the treatment responding information can be performed based on image analytic, machine learning, deep learning, or data computing. For example, the step of determining the treatment responding information can comprise analyzing the second medical image data, wherein preferably a tumor target information is determined. The tumor target information is preferably obtained based on the second medical image data, especially the images comprised by the second medical image data. The tumor target information comprises for example a volume, position, shape, physical, chemical, biological, medical and/or histological information of the tumor target.

The supplementary data comprise the first medical image data, medical image data acquired between the first and second medical image data, the initial treatment plan and/or an initial tumor target information. The supplementary data comprise especially data and/or information, which allows a comparison of the tumor target and/or tumor target information based on the first medical image data and/or before applying the radiotherapy treatment with the tumor target and/or tumor target information based on the second medical image data.

The treatment continuation information is determined based on the treatment responding information and on the initial treatment plan. The treatment continuation information comprises an information and/or suggestion for a further treatment of the patient and/or further radiotherapy of the patient. For example, a suggestion to change the radiotherapy of the patient by amending the initial treatment plan, especially amending it in a suggested way, or to continue the radiotherapy with the initial treatment plan is determined as treatment continuation information. Optionally, determining the treatment continuation information comprises determining a treatment plan for the further treatment and/or further radiotherapy, in particular determining the treatment plan based on the initial treatment plan, the treatment responding information and/or the treatment continuation information. Determining the treatment plans means especially adapting, modifying and/or changing the initial treatment plan. The initial treatment plan is especially adapted and/or adjusted based on changes of the tumor target during the first treatment period. For example, during the first treatment period the tumor target has reduced (e.g., volume), wherein the initial treatment plan is adapted to this, e.g., by reducing the dose or beam field size. Particularly, when the tumor target has not responded to radiotherapy during the first treatment period and/or has increased its size, the generated treatment plan reflects this and sets for example a higher dose or suggest additional treatments. The step determining the treatment continuation information comprises for example a suggestion for an adaption of the initial treatment plan, an alteration of a course of treatment, a boost of treatment and/or a cessation of treatment.

The determined treatment continuation information and/or the determined treatment plan is provided, especially provided to a data storage, a cloud, a radiotherapy system. In particular, the treatment continuation information and/or the treatment plan is provided to a user, e.g., a doctor, for cross-checking, adapting, executing and/or confirming the suggestion, the treatment continuation information and/or determined treatment plan. The determined treatment plan is for example provided to the user by a display or monitor. Preferably, the determined treatment plan is provided to the radiotherapy system for applying and/or implementing it. For example, the generated treatment plan is performed by the radiotherapy system and/or applied to the patient.

The invention is based on the idea to use second medical image data to determine a treatment response information, which shows the effectiveness of the radiotherapy treatment according to the initial treatment plan, wherein based on this treatment response information and the initial treatment plan a treatment continuation information and/or (new or adapted) treatment plan is generated.

In particular, the step determining a treatment responding information comprises determining a change of a characteristic of the tumor target and/or nearby organ. The step can also comprise determining a plurality of changes of a characteristic of the tumor target. The change of the characteristic is particularly determined based on the first and second medical image data, e.g., by comparing the first and second medical image data. For example, the step determining the treatment responding information comprises determining a characteristic of the tumor target in the first and in the second medical image data, wherein the change of the characteristic is determined based on the determined characteristic of the tumor target in the first medical image data and on the determined characteristic of the tumor target in the second medical image data, e.g., by comparing them and/or calculating the difference.

The characteristic of tumor target can be configured as or comprising a contour of the target tumor, a volume of the target tumor, an apparent diffusion coefficient of the target tumor, a perfusion of the target tumor, a metabolism of the target tumor, a tumor marker, a position, an orientation, a motion of the target tumor and/or an appearance of oedema. The contour can be a three-dimensional contour, e.g., shape, shell, or surface. The contour can be a two-dimensional contour, an outline or projection to a plane. The volume can be configured as an absolute value, e.g., a volume measure, or be configured as 3D-Model comprising volume measure and shape. The apparent diffusion coefficient (ADC) is a measure of the magnitude of diffusion (of water molecules) within tissue, and is commonly clinically calculated using MRI with diffusion-weighted imaging (DWI). The perfusion is especially understood as the passage of fluid through the lymphatic system or blood vessels to an organ or a tissue. The perfusion can be determined and/or used as qualitative or quantitative perfusion. The perfusion is for example determined based on Microspheres, CT images, MR images, PET images or nuclear medicine data. The tumor metabolism can be determined based on real-time metabolic flux, e.g., can be measured non-invasively using hyperpolarized magnetic resonance spectroscopy in cancer spheroids as well as tumor biopsies. The tumor markers and/or the change of the tumor markers (e.g., positive, or negative) can be determined based on biopsy or laboratory diagnostic, e.g., using as tumor markers molecules in serum that are elevated in various malignancies and are often used to monitor treatment response as well as alert for potential progressive disease when in remission. Commonly used markers include AFP (alpha fetoprotein), beta-hCG, CA 15-3, CA 19-9, CA 27-29, CA-125, CEA, chromogranin A, human epididymis protein-4 (HE4), lactate dehydrogenase (LDH), neuron-specific enolase (NSE), PSA (prostate specific antigen), S100, vimentin. The position can be an absolute position, e.g., relative to a world coordinate system or relative to a body system. The position is especially a position in three dimensions. The orientation is preferably an orientation in three-dimensional space. The orientation can be absolute or relative, e.g., relative to an organ or body mark. The motion is preferably a motion in two or three dimensions. The motion can be determined as a vectorial motion. The motion can be determined and/or used as direction of the motion (e.g., direction vector) and/or function of time (e.g., v̅(t)), for example to describe a cyclic motion.

In a preferred embodiment of the invention the step determining a treatment responding information comprises determining a map of the change of a characteristic of the tumor target. The map can be configured as a two- or three-dimensional map. Preferably the map is configured as a heatmap. The step determining a treatment responding information comprises in particular providing the map to a user and/or to display the map to a user on a display or monitor. The map is in particular comprising a model (2D or 3D) of the tumor target, wherein the change of a characteristic of the tumor target is indicated or shown. For example, the map comprises a three-dimensional image or model of tumor target, wherein the change of the size or shape of the tumor target is indicated in the image or model, e.g., by coloring, wherein in particular also a change in the apparent diffusion coefficient and/or motion can be indicated in the image or model. The map can for example be used to support the user in the verification, confirmation, checking and/or adaption of the determined treatment plan.

Preferably, the method comprises as a step an acquiring of the second medical image data of the patient with a medical imaging device. The step of acquiring the second medical image data is prior to the step of determining the change of the characteristic of the tumor target, in particular the step acquiring the second medical image data is comprised by the step receiving second medical image data. Especially, the step acquiring the second medical image data can comprise acquiring second medical image data with a plurality of medical image devices, e.g., acquiring CT image data with a computed tomography scanner and acquiring MR image data with a magnetic resonance tomography scanner. The acquiring of the second medical image data can be performed with a magnetic resonance tomography scanner, a computed tomography scanner, positron emission tomography scanner, an ultrasound scanner and/or an X-ray scanner as medical imaging device.

In particular, the step determining the treatment responding information is performed by the medical imaging device and/or while the patient is positioned for the acquiring of the second medical image data. Determining the treatment responding information is in particular done prior positioning the patient for the radiotherapy and/or to apply the treatment plan to the patient. In other words, the method is based and/or carried out by two separate systems, units, devices and/or at separate local areas (e.g., rooms), wherein for example one device is the medical image device and the other is a radiotherapy unit for applying the radiotherapy according to the treatment plan. Especially, determining the treatment responding information and determining the treatment plan or performing the treatment are carried out at separate devices, units, systems, or locations. The system, device, unit, or location, especially the medical imaging devices provides the determined treatment responding information to the step determining the treatment plan, especially to the device, unit, system, or location carrying out the step determining the treatment plan. The step determining the treatment plan is preferably performed by a radiotherapy unit and/or at a treatment location, wherein the radiotherapy unit is separated and/or spatially separate from the medical imaging device and/or from the location for acquiring the second medical image data. For example, the second medical image data are acquired with the medical imaging device in a first room, wherein the radiotherapy, determining and/or performing of the treatment plan is carried out in a different room. The inventor recognized splitting the performance of the steps of the method to two separate devices increases the patient throughput.

According to an optional embodiment of the invention, the step determining the treatment responding information comprises triggering an acquiring of further second medical image data. The acquiring of further second medical images is triggered when a certain change of a tumor characteristic is determined based on the already acquired second medical image data. For example, the second medical image data are acquired with an MRI scanner using a sequence optimized for data acquisition without contrast agent, wherein based on this image data an increasing in the volume of the tumor target is determined, wherein the based on the detected increase of the volume an acquiring of further medical image data using a different sequence, contrast agent or a different kind of scanner is triggered. This allows a data, recourse and time optimized determination of the treatment plan and concerning medical image data.

Preferably, the step determining the treatment continuation information and/or determining the treatment plan comprises applying a trained function to the treatment responding information and the initial treatment plan. The trained function is in particular configured as or based on a machine learning, especially deep learning, algorithm, or method. The trained function is preferably based on a neuronal network, e.g., a convolutional neuronal network. The function is trained for determining a treatment continuation information and/or for determining a treatment plan based on an initial treatment plan and treatment responding information. Applying the trained function to the treatment responding information and the initial treatment plan determines and/or generates the treatment continuation information, especially determines the suggestion or the treatment plan, e.g., determines it as output data and/or as feature vector. In particular, applying the trained function to the initial treatment plan determines a probability for the accuracy of the determined treatment continuation information and/or the determined treatment plan.

Optionally, the step determining the treatment response information comprises applying a further trained function to the second medical image data and the supplementary data. The further trained function is in particular configured as or based on a machine learning, especially deep learning, algorithm or method. The further trained function is preferably based on a neuronal network, e.g., a convolutional neuronal network. The further function is trained for determining a treatment responding information based on second medical image data and on supplementary data. Applying the further trained function to the second medical image data and the supplementary data determines and/or generates the treatment responding information, e.g., as output data and/or as feature vector. In particular, applying the further trained function to the second medical image data and the supplementary data determines a probability for the accuracy of the determined treatment responding information.

Especially, the step determining the treatment continuation information comprises determining a comparison result. The comparison result is determined based on comparing the determined treatment responding information, particularly the change of the characteristic of the tumor target, with a threshold. E.g., it is determined whether the change of a characteristic of the tumor target exceeds or falls below a threshold, wherein for different characteristics of the tumor target different thresholds can be used. In particular, the comparing can comprise checking if the change of the characteristic of the tumor target lies within an interval or range, e.g., if the change is not too big and not too small, e.g., in a forecasted range. In particular, the initial treatment plan is adapted, modified and/or changed as the treatment plan, when the comparison result does not fulfill a preset rule, e.g., when the change exceeds the threshold or when the change is not in the forecasted range. In particular, the initial treatment plan is kept and/or determined as treatment plan, when the comparison result fulfills the preset rule, e.g., when the change is not exceeding the threshold or when the change is in the forecasted range.

In an embodiment of the invention the method comprises the step providing additional information data for the patient, wherein the additional information data comprise a patient file, a patient reported outcome score, a patient self-report and/or biopsy information. The additional information data are for example provided by a cloud or patient data storage. The step determining the treatment continuation information, especially determining the treatment plan, is based on the additional information. In other words, the adaption of the initial treatment plan to determined treatment plan is carried out under consideration of the additional information data. For example, the trained function can be trained to determine the treatment plan based on the treatment responding information, the initial treatment plan and the additional information data.

Furthermore, the invention discloses a system for determining a treatment continuation information for a radiotherapy of a tumor target of a patient, comprising an interface, a responding information determining unit, a continuation information determining unit and a providing unit. The system is configured to carry out and/or to implement the method according to the invention.

The interface is configured to receive an initial treatment plan for a radiotherapy of the patient, wherein the initial treatment plan is based on first medical image data of the patient, wherein the first medical image data comprise the tumor target. The interface is for example configured to connect the system, the responding information determining unit, the continuation information determining unit and/or the providing unit with a storage device, e.g., a cloud, or a data source. The interface is in particular configured as a user interface.

The interface is configured to receive second medical image data of the patient, wherein the second medical image data comprise the tumor target, wherein the second medical image data are acquired a first treatment time period after the first medical image data, wherein the first treatment time period comprises at least one application of a radiotherapy to the patient based on the initial treatment plan. The interface is for example configured for a connection with a medical imaging device, e.g., with a CT scanner, an MRI scanner, or a PET scanner.

The responding information determining unit is preferably configured as a processor or comprises a processor. The responding information determining unit is preferably comprised by the medical imaging device and/or located in the same area or room. The responding information determining unit is configured to determine the treatment responding information based on the second medical image data and supplementary data, wherein the supplementary data comprise the first medical image data, medical image data acquired between the first and second medical image data, the initial treatment plan and/or an initial tumor target information. Especially, the responding information determining unit is configured to process and/or implement the step of determining the treatment responding information of the inventive method.

The continuation information determining unit is preferably configured as a processor or comprises a processor. The continuation information determining unit is in particular comprised by a radiotherapy system, e.g., a LINAC, and/or located in the same area or room as the radiotherapy system. The continuation information determining unit is configured to determine the treatment continuation information, especially to determine a treatment plan, based on the treatment responding information and the initial treatment plan.

The providing unit is configured to provide the treatment continuation information and/or the treatment plan. The providing unit is for example an interface to a storage device, e.g., to a cloud, and/or configured as a user interface. The providing unit preferably provides the determined treatment continuation information and/or the treatment plan to the radiotherapy system, e.g., to carry out the radiotherapy to the patient. Optionally, the providing unit is configured to provide, especially to display or show, the treatment continuation information and/or the treatment plan to a user, e.g., a medical doctor to be checked, verified, or amended by the user.

The system enables detection and more accurate treatment of a tumor target in radiotherapy patients. In radiotherapy, 3D X-Ray initial images are acquired to plan a treatment process. Subsequent images (of which many dozen may be taken) may be used to position a patient but are typically not used to re-plan and refine the treatment process, nor are subsequent images used to incrementally or in ensemble, determine anatomical changes (substantive or otherwise) with respect to an initial planning image. The system provides updated and replanned treatment based on second medical image data and the initial treatment plan.

A processor as used herein is especially a device and/or set of machine-readable instructions for performing tasks. A processor comprises any one or combination of, hardware, firmware, and/or software. A processor acts upon information by manipulating, analyzing, modifying, converting, or transmitting information for use by an executable procedure or an information device, and/or by routing the information to an output device. A processor may use or comprise the capabilities of a controller or microprocessor, for example. A processor may be coupled (electrically and/or as comprising executable components) with any other processor enabling interaction and/or communication there-between. A display processor or generator is a known element comprising electronic circuitry or software or a combination of both for generating display images or portions thereof. A user interface comprises one or more display images enabling user interaction with a processor or other device.

An executable application, as used herein, comprises preferably code or machine-readable instructions for conditioning the processor to implement predetermined functions, such as those of an operating system, a context data acquisition system or other information processing system, for example, in response to user command or input. An executable procedure is a segment of code or machine-readable instruction, subroutine, or other distinct section of code or portion of an executable application for performing one or more processes. These processes may include receiving input data and/or parameters, performing operations on received input data and/or performing functions in response to received input parameters, and providing resulting output data and/or parameters. A user interface (UI), as used herein, comprises one or more display images, generated by a display processor and enabling user interaction with a processor or other device and associated data acquisition and processing functions.

The UI also includes an executable procedure or executable application. The executable procedure or executable application conditions the display processor to generate signals representing the UI display images. These signals are supplied to a display device which displays the image for viewing by the user. The executable procedure or executable application further receives signals from user input devices, such as a keyboard, mouse, light pen, touch screen or any other means allowing a user to provide data to a processor. The processor, under control of an executable procedure or executable application, manipulates the UI display images in response to signals received from the input devices. In this way, the user interacts with the display image using the input devices, enabling user interaction with the processor or other device.

In a preferred embodiment, the system further comprises a medical imaging device. Alternatively, the system is connected to the medical imaging device, e.g., via the interface, especially connected to exchange data and/or to trigger processes. The medical imaging device is configured to acquire the second medical image data of the patient. The medical imaging device is configured as or comprises a magnetic resonance tomography, a computed tomography, positron emission tomography, ultrasound scanner and/or X-ray scanner. The medical imaging device is configured to provide the second medical image data to the responding information determining unit and/or to the system. In particular, the system, especially the responding information determining unit, is configured to trigger the medical imaging system to acquire the second medical image data and/or to acquire the further second medical image data. Optionally, the medical imaging device comprises the responding information determining unit and/or is configured to determine the treatment responding information based on the second medical image data and supplementary data. This is especially based on the insight, to split imaging, image processing and treatment performance or treatment plan determining to have an optimized patient throughput.

In particular, the system further comprises a radiotherapy system configured to execute and/or apply the treatment plan. The radiotherapy system is for example configured as a LINAC (Linear accelerator). Alternatively, the system is connected to the radiotherapy system, e.g., for data exchange and/or for determining or providing the treatment plan. The radiotherapy unit is preferably physically and/or spatially separated from the medical imaging device, e.g., in a different room. Optionally, the radiotherapy system comprises the treatment plan determining unit and/or is configured to acquire the step determining the treatment plan according to the invention.

According to a third aspect the invention relates to a computer program comprising instructions which, when the program is executed by a computer, processor and/or system according to the invention, cause the system to carry out the method according to the invention and its aspects.

According to a fourth aspect the invention relates to a computer-readable medium comprising instructions which, when executed by a computer, processor and/or system according to the invention, cause the system to carry out the method according to the invention and its aspects.

The realization of the invention by a computer program product and/or a computer-readable medium has the advantage that already existing systems can be easily adopted by software updates to work as proposed by the invention.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

The properties, features and advantages of this invention described above, as well as the manner they are achieved, become clearer and more understandable in the light of the following description and embodiments, which will be described in detail in the context of the drawings. This following description does not limit the invention on the contained embodiments. Same components or parts can be labeled with the same reference signs in different figures. In general, the figures are not for scale. In the following:
- Figure 1: shows a system for determining a treatment continuation information for a radiotherapy.
- Figure 2: shows a second example of a system for determining a treatment continuation information.
- Figure 3: shows a flowchart of a method for determining a treatment continuation information.

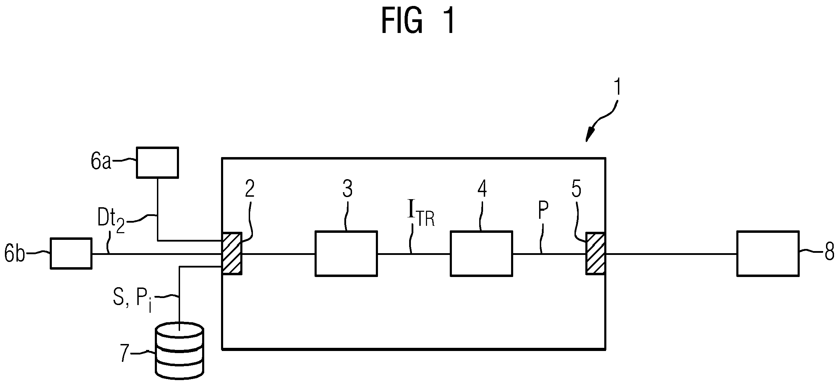

FIG. 1 shows a system 1 for determining a treatment continuation information, wherein the treatment continuation information comprises a suggestion for continuing the radiotherapy of the patient with a treatment plan P, which can be the equal or different to an initial treatment plan Pi. The system 1 includes an interface 2, a responding information determining unit 3, a continuation information determining unit 4 and a providing unit 5. The responding information determining unit 3 and the continuation information determining unit 4 can be aggregated and/or configured as one unit, especially as a processing device (e.g., workstations or portable devices such as notebooks, Personal Digital Assistants, phones). The interface 2 is configured for coupling with at least one medical imaging device 6a, 6b and/or with a data storage 7, e.g., a cloud. The interface 2 is especially configured for cabled or wireless connection and/or for a data exchange. The medical imaging devices 6a, b provides the system 1, especially the responding information determining unit 3, second medical image data Dₜ₂. The storage 7 is configured for providing supplementary data S, first medical image data Dₜ₁ and/or an initial treatment plan Pᵢ. The medical imaging device 6a, b is for example a MR (magnetic resonance), CT scan, X-ray, or Ultra-sound device.

The responding information determining unit 3 is configured to determine a treatment responding information I_{TR} based on the second medical image data Dₜ₂ and based on supplementary data S. The supplementary data S can comprise the first medical image data Dₜ₁, a tumor target characteristic determined based on the first medical image data Dₜ₂ (initial tumor target information) the initial treatment plan Pᵢ and/or information concerning the tumor target at a time between and/or including acquisition of the first and the second medical image data. For example, the responding information determining unit 3 determines an initial tumor target characteristic based on the first medical image data Dₜ₁ and determines a tumor target characteristic based on the second medical image data Dₜ₂, wherein the responding information determining unit 3 determines the treatment responding information I_{TR} based on the determined initial tumor target characteristic and the tumor target characteristic, e.g., based on a comparison. The responding information determining unit 3 provides the determined treatment responding information I_{TR} to the plan determining unit 4.

The continuation information determining unit 4 is configured to determine a suggestion, to determine a treatment plan P, especially to suggest a decision for continuation of the radiotherapy of the patient, based on the initial treatment plan Pᵢ and the determined treatment responding information I_{TR}. The continuation information determining unit 4 is for example configured to suggest or execute an adaption, change and/or amendment of the initial treatment plan Pᵢ for a further and/or continuation of the radiotherapy of the patient. In particular the treatment continuation information can comprise generating the treatment plan P as an amendment of the initial plan Pi, when the treatment responding information I_{TR} indicates that the treatment is already responding and/or the treatment does not respond as forecasted. E.g., the continuation information determining unit 4 uses the initial treatment plan Pᵢ as the determined treatment plan P, when the tumor target seems to respond as forecasted (shrinking can already be forecasted), wherein the p continuation information determining unit 4 adapts or suggest the adaption of the initial treatment plan Pᵢ, e.g., by adapting radiotherapy parameters, when the tumor target has not responded to the radiotherapy or responded more than forecasted. The continuation information determining unit 4 provides the determined treatment plan P or provides the determined treatment continuation information to the providing unit 5. The providing unit 5 provides the treatment plan P and/or the treatment continuation information, especially the suggestion, to a radiotherapy system 8 or a user, e.g., a doctor or radiological physicist.

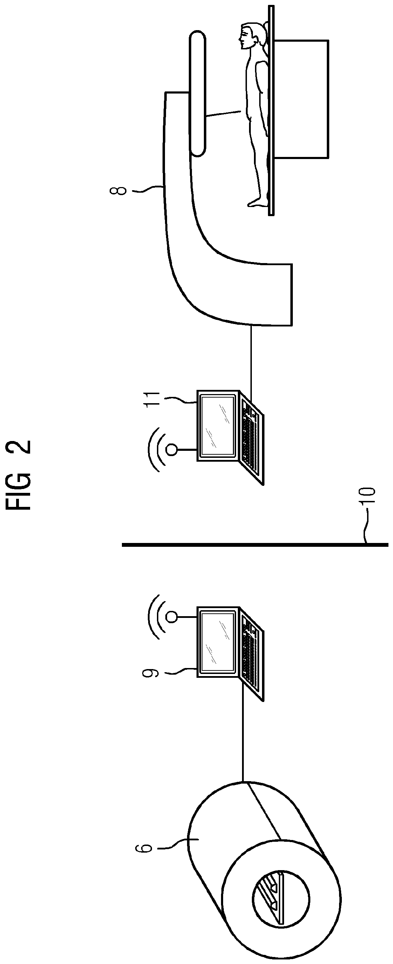

Figure 2 shows another example of a system 1 for determining a treatment continuation information, especially for determining the treatment plan P for a radiotherapy of patient. The system 1 is based on the embodiment of the system 1 of figure 1. According to the second example, the system 1 comprises the medical imaging device 6 and the radiotherapy system 8. The medical imaging device 6 is for example a MRI, CTI or PET scanner and connected with a processor 9, e.g., a computing unit. The processor 9, especially as computing unit, can be configured for image processing of second medical images, provided by the medical imaging device 6. For example, the processor 9 is configured for providing the second medical image data to a user for diagnosis or checks.

The processor 9, especially as computing unit, comprises the responding determining unit 3. In other words, the unit for analysis and presenting the second medical image data Dₜ₂ is also configured for determining the treatment responding information I_{TR}.

The radiotherapy system 8 is spaced apart from the medical imaging device 6. E.g., the radiotherapy system 8 and the medical imaging device 6 are separated by wall 10 and/or located in different rooms. The radiotherapy system 8 comprises a or relates to a processor 11, e.g., a computing unit. The processor 11, especially as computing unit, can be configured for setting up a treatment plan and/or for managing (e.g., starting, adapting, stopping) the radiotherapy system 8 and/or radiotherapy treatment. The processor 9 and the processor 11 are connected for data transfer, e.g., using a wireless data connection 12. The processor 9 is configured to provide the determined treatment responding information I_{TR} to the processor 11. The processor 11 is comprises the continuation information determining unit 4 and is configured to determine the treatment continuation information, especially to determine the treatment plan P, based on the provided treatment responding information I_{TR} and the initial treatment plan Pᵢ. The initial treatment plan Pᵢ can be deposed on the processor 11 or be provided by the processor 9. The processor 11 and/or the continuation information determining unit 4 provides the treatment continuation information, especially the treatment plan P, to the radiotherapy system 8, wherein the radiotherapy system executes the treatment plan P or provides the treatment continuation information to a user for verification and/or amending the radiotherapy based on the treatment continuation information.

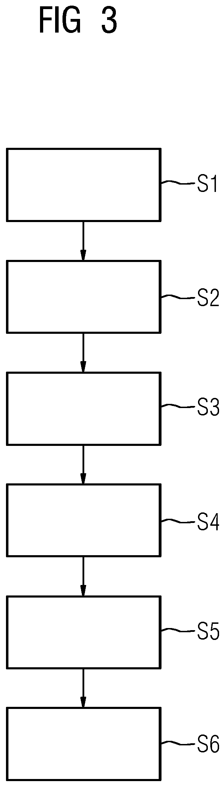

Figure 3 shows a flowchart of a method for determining a treatment continuation information, especially for determining the treatment plan P as part of the treatment continuation information.

In step S1 a medical imaging device 6 acquires second medical image data Dₜ₂. Prior to acquiring the second medical image data Dₜ₂ first medical imaging data Dₜ₁ were acquired, especially by the same or different medical imaging device 6, wherein the acquisition of the first medical imaging data Dₜ₁ is optionally part of the method. In step S2 the second medical image data Dₜ₂ are received, e.g., by the system 1 for determining the treatment plan P and/or by the responding information determining unit 3.

In step S3 an initial treatment plan Pᵢ for a radiotherapy of a patient is received. The treatment plan Pᵢ can be provided by a storage 7 or a radiotherapy system 8. The initial treatment plan Pᵢ is based on the first medical image data Dₜ₁. The initial treatment plan is provided to the responding information determining unit 3.

In step S4 treatment responding information I_{TR} are determined, especially by the responding information determining unit 3. The treatment responding information I_{TR} is determined based on the second medical image data Dₜ₂ and supplementary data S, e.g., comprising the first medical image data Dₜ₁ and/or the initial treatment plan Pᵢ.

In step S5 the treatment continuation information is determined. The step determining the treatment continuation information comprises determining the treatment plan P. The step S5 determining the treatment continuation information especially by the continuation information determining unit 4. The treatment plan P is determined based on the determined treatment responding information I_{TR} and the initial treatment plan Pᵢ, e.g., determined by adapting the initial treatment plan Pᵢ based on the response of the tumor target to the already carried out radiotherapy treatment. In Step S6 the determined treatment continuation information and/or the determined treatment plan P is provided to a radiotherapy system 8 for applying the radiotherapy to the patient based on the determined and adapted treatment plan P.

## Claims

1. Method for determining a treatment continuation information for a radiotherapy of a tumor target of a patient, comprising the steps:
- Receiving (S3) an initial treatment plan (Pᵢ) for a radiotherapy of the patient, wherein the initial treatment plan (Pᵢ) is based on first medical image data (Dₜ₁) of tumor target,
- Receiving (S2) second medical image data (Dₜ₂) of the tumor target, wherein the second medical image data (Dₜ₂) are acquired a first treatment time period after acquiring the first medical image data (Dₜ₁),
- Determining (S4) a treatment responding information (I_{TR}) based on the second medical image data (Dₜ₂) and on supplementary data (S), wherein the supplementary data (S) comprise the first medical image data (Dₜ₁), medical image data acquired between the first and second medical image data (Dₜ₁, Dₜ₂), the initial treatment plan (Pᵢ) and/or an initial tumor target information,
- Determining (S5) the treatment continuation information based on the treatment responding information (I_{TR}) and on the initial treatment plan (Pᵢ), wherein the treatment continuation information comprises an information and/or suggestion for a further treatment of the patient and/or further radiotherapy of the patient,
- Providing (S6) the treatment continuation information.

2. Method according to claim 1, wherein the step determining (S5) the treatment continuation information comprises determining a suggestion to continue the treatment and/or radiotherapy of the patient by adapting the initial treatment plan, by continuing with the initial treatment plan, by alteration of a course of treatment, by a boost of the treatment and/or a cessation of the treatment.

3. Method according to claim 1 or 2, wherein the step determining (S4) a treatment responding (I_{TR}) comprises determining a change of a characteristic of the tumor target, wherein the characteristic comprises a contour of the target tumor, a volume of the target tumor, an apparent diffusion coefficient of the target tumor, a perfusion of the target tumor, a metabolism of the target tumor, a tumor marker, a motion of the target tumor and/or an appearance of oedema.

4. Method according to claim 3, wherein the step determining (S4) a treatment responding information (I_{TR}) comprises determining a map of the change of a characteristic of the tumor target.

5. Method according to one of the previous claims, further comprising:
- Acquiring the second medical image data (Dₜ₂) with a medical imaging device (6, 6a, b), wherein the medical imaging device (6, 6a, b) comprises a MRI scanner, CTI scanner, PET scanner, an ultrasound scanner and/or an X-ray scanner.

6. Method according to claim 5, wherein the step determining (S4) a treatment responding information (I_{TR}) is performed by the medical imaging device (6, 6a, b) and/or while the patient is positioned for the acquiring of the second medical image data (Dₜ₂) .

7. Method according to claim 6, wherein the step determining (S4) a treatment responding information (I_{TR}) comprises triggering an acquiring of further second medical image data, when a certain change of a tumor characteristic is determined based on the acquired second medical image data (Dₜ₂).

8. Method according to one of the previous claims, wherein the step determining (S5) the treatment continuation information comprises applying a trained function to the treatment responding information (I_{TR}) and to the initial treatment plan (Pᵢ), wherein applying the trained function to the treatment responding information (I_{TR}) and to the initial treatment plan (Pᵢ) determines the treatment continuation information.

9. Method according to one of the previous claims, wherein the step determining (S5) the treatment continuation information comprises determining a comparison result by comparing the treatment responding information (I_{TR}) and/or the change of the characteristic of the tumor target with a threshold, wherein as the treatment continuation information a suggestion to adapt, modify and/or change the initial treatment plan (Pi) is determined, when the comparison result does not fulfill a preset rule, wherein as the treatment continuation information a suggestion to continue the treatment with the initial treatment plan (Pᵢ), when the comparison result fulfills the preset rule.

10. Method according to one of the previous claims, further comprising:
- providing additional information data for the patient, wherein the additional information data comprise a patient file, a patient reported outcome score, a patient self-report and/or biopsy information, wherein determining of the treatment continuation information is based on the additional information.

11. Method according to one of the previous claims, wherein the step determining (S5) the treatment continuation information is performed by a radiotherapy system (8) and/or at a treatment location, wherein the radiotherapy system (8) is separated and/or spatially separate from the medical imaging device (6, 6a, b) and/or from the location for acquiring the second medical image data (Dₜ₂).

12. System (1) for determining a treatment continuation information for a radiotherapy of a tumor target of a patient, comprising an interface (2), a responding information determining unit (3), a plan determining unit (4) and a providing unit (5):
wherein the interface (2) is configured to receive an initial treatment plan (Pᵢ) for a radiotherapy of the patient, wherein the initial treatment plan (Pᵢ) is based on first medical image data (Dₜ₁) of the tumor target,
wherein the interface (2) is configured to receive second medical image data (Dₜ₂) of the tumor target, wherein the second medical image data (Dₜ₂) are acquired a first treatment time period after acquiring the first medical image data (Dₜ₁), wherein the first treatment time period comprises at least one application of a radiotherapy to the patient based on the initial treatment plan (Pᵢ),
wherein the responding information determining unit (3) is configured to determine the treatment responding information (I_{TR}) based on the second medical image data (Dₜ₂) and supplementary data (S), wherein the supplementary data (S) comprise the first medical image data (Dₜ₁), medical image data acquired between the first and second medical image data- (Dₜ₁, Dₜ₂), the initial treatment plan (Pᵢ) and/or an initial tumor target information,
wherein the plan determining unit (4) is configured to determine the treatment continuation information based on the treatment responding information (I_{TR}) and the initial treatment plan (Pᵢ),
wherein the providing unit (5) is configured to provide the treatment continuation information.

13. System (1) according to claim 12, further comprising a medical imaging device (6, 6a, b), wherein the medical imaging device (6, 6a, b) is configured to acquire the second medical image data (Dₜ₂) of the patient, wherein the medical imaging device (6, 6a, b) comprises a MRI scanner, CTI scanner, PET scanner, ultrasound scanner and/or X-ray scanner, wherein the medical imaging device (6, 6a, b) is configured to provide the second medical image data (Dₜ₂) and/or to determine the treatment responding information (I_{TR}) based on the second medical image data (Dₜ₂) and supplementary data (S) .

14. System (1) according to claim 13, further comprising a radiotherapy system (8) configured to execute and/or apply the treatment plan (P), initial treatment plan (Pi) and/or to determine, execute, implement and/or display the treatment continuation information, wherein the radiotherapy unit (8) is physically and/or spatially separated from the medical imaging device (6, 6a, b)

15. Computer program comprising instructions which, when the program is executed by a computer, processor and/or system (1) according to one of the claims 12 to 14, cause the computer, processor or system (1) to carry out the method according to one of the claims 1 to 11.

16. Computer-readable medium comprising instructions which, when executed by a computer, processor and/or system (1) according to one of the claims 12 to 14, cause the computer, processor or system (1) to carry out the method according to one of the claims 1 to 11.
